# EUROPEAN PATENT SPECIFICATION

(11) **EP 1 691 607 B1**
(45) Date of publication and mention of the grant of the patent: **18.04.2007**
(21) Application number: 04803437.5
(22) Date of filing: 02.12.2004
(51) Int. Cl.: A01N 37/52

(54) **THE USE OF N-ARYLHYDRAZINE DERIVATIVES FOR COMBATING NON-CROP PESTS**
VERWENDUNG VON N-ARYLHYDRAZINDERIVATEN FÜR DIE BEKÄMPFUNG VON SCHÄDLINGEN , WELCHE KULTURPFLANZEN NICHT ANGREIFEN
UTILISATIONS DES DERIVES DE LA N-ARYLHYDRAZINE POUR COMBATTRE LES ORGANISMES NUISIBLES N'ATTAQUANT PAS LES CULTURES

(30) Priority: 04.12.2003 US 526609 P
(43) Date of publication of application: 23.08.2006
(73) Proprietor: BASF Aktiengesellschaft, 67056 Ludwigshafen (DE)
(72) Inventor: VON DEYN, Wolfgang, 67435 Neustadt (DE); OLOUMI-SADEGHI, Hassan, Raleigh, NC 27614 (US); KUHN, David, G., Apex, NC 27502 (US); ARMES, Nigel, Raleigh, NC 27613 (US); KORADIN, Christopher, 67067 Ludwigshafen (DE)
(86) International application number: PCT/EP2004/013687
(87) International publication number: WO 2005/053403

(56) References cited:
- EP-A- 0 604 798
- DATABASE CA [Online] CHEMICAL ABSTRACTS SERVICE, COLUMBUS, OHIO, US; KUHN, D. G. ET AL KUHN, D. G. ET AL: "Cycloalkyl-substituted amidrazones: a novel class of insect control agents Cycloalkyl-substituted amidrazones: a novel class of insect control agents" XP002330111 retrieved from STN Database accession no. 1998:294837 & ACS SYMPOSIUM SERIES , 686(SYNTHESIS AND CHEMISTRY OF AGROCHEMICALS V), 185-193 CODEN: ACSMC8; ISSN: 0097-6156 ACS SYMPOSIUM SERIES , 686(SYNTHESIS AND CHEMISTRY OF AGROCHEMICALS V), 185-193 CODEN: ACSMC8; ISSN: 0097-6156, 1998, cited in the application
- DATABASE CA [Online] CHEMICAL ABSTRACTS SERVICE, COLUMBUS, OHIO, US; FURCH, J. A. ET AL FURCH, J. A. ET AL: "Amidrazones: a new class of coleopteran insecticides Amidrazones: a new class of coleopteran insecticides" XP002330112 retrieved from STN Database accession no. 1998:294826 & ACS SYMPOSIUM SERIES , 686(SYNTHESIS AND CHEMISTRY OF AGROCHEMICALS V), 178-184 CODEN: ACSMC8; ISSN: 0097-6156 ACS SYMPOSIUM SERIES , 686(SYNTHESIS AND CHEMISTRY OF AGROCHEMICALS V), 178-184 CODEN: ACSMC8; ISSN: 0097-6156, 1998, cited in the application

## Description

The present invention relates to the use of hydrazine derivatives of formula I: 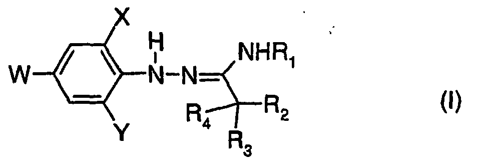 wherein
- W: is chlorine or trifluoromethyl;
- X and Y: are each independently chlorine or bromine;
- R¹: is C₁-C₆-alkyl, C₃-C₆-alkenyl, C₃-C₆-alkynyl, or C₃-C₆-cycloalkyl which may be substituted with 1 to 3 halogen atoms, or C₂-C₄-alkyl which is substituted by C₁-C₄-alkoxy;
- R² and R³: are C₁-C₆-alkyl or may be taken together to form C₃-C₆-cycloalkyl which may be unsubstituted or substituted by 1 to 3 halogen atoms;
- R⁴: is hydrogen or C₁-C₆-alkyl,
or the enantiomers or salts thereof,
for combating non-crop pests.

The invention also relates to a method for controlling non-crop pests comprising contacting the pests or their food supply, habitat, breeding grounds or their locus with a pesticidally effective amount of a compound of formula I.

The invention also relates to compositions containing compounds of formula I for in pesticidally effective amounts for controlling non-crop pests.

The invention further relates to the use of compounds of formula I for the protection of non-living organic materials against non-crop pests.

Typical problems arising with the use of presently available non-crop pest control agents such as pyrethroids are e.g. resistance of pests or unfavorable environmental or toxicological properties. Another problem encountered concerns the need to have available non-crop pest control agents which are effective against a broad spectrum of non-crop pests. Accordingly, there is a need to provide new and improved non-crop pest control agents that overcome these problems.

It is therefore an object of the present invention to provide new non-crop pest control agents, preferably exhibiting an enhanced pesticidal spectrum of action.

We have found that these objects are achieved by use of compounds of formula I and compositions comprising them.

Non-crop pests are pests of the classes Chilopoda and Diplopoda and of the orders Isoptera, Diptera, Blattaria (Blattodea), Dermaptera, Hemiptera, Hymenoptera, Orthoptera, Siphonaptera, Thysanura, Phthiraptera, Araneida, Parasitiformes and Acaridida.

The compounds of the formula I are especially suitable for efficiently combating the following pests:
centipedes (Chilopoda), e.g. *Scutigera coleoptrata,*
millipedes (Diplopoda), e.g. *Narceus spp.,*
spiders (Araneida), e.g. *Latrodectus mactans,* and *Loxosceles reclusa,*
scabies (Acaridida): e.g. *sarcoptes sp,*
ticks and parasitic mites (Parasitiformes): ticks (Ixodida), e.g. *Ixodes scapularis, Ixodes holocyclus, Ixodes pacificus, Rhiphicephalus sanguineus, Dermacentor andersoni, Dermacentor variabilis, Amblyomma americanum, Ambryomma maculatum, Ornithodorus hermsi, Ornithodorus turicata* and parasitic mites (Mesostigmata), e.g. *Ornithonyssus bacoti* and *Dermanyssus gallinae,*
termites (Isoptera), e.g. *Calotermes flavicollis, Leucotermes flavipes, Heterotermes aureus, Reticulitermes flavipes, Reticulitermes virginicus, Reticulitermes lucifugus, Termes natalensis,* and *Coptotermes formosanus,*
cockroaches (Blattaria - Blattodea), e.g. *Blattella germanica, Blattella asahinae, Periplaneta americana, Periplaneta japonica, Periplaneta brunnea, Periplaneta fuligginosa, Periplaneta australasiae,* and *Blatta orientalis,*
flies, mosquitoes (Diptera), e.g. *Aedes aegypti, Aedes albopictus, Aedes vexans, Anastrepha ludens, Anopheles maculipennis, Anopheles crucians, Anopheles albimanus, Anopheles gambiae, Anopheles freeborni, Anopheles leucosphyrus, Anopheles minimus, Anopheles quadrimaculatus, Calliphora vicina, Chrysomya bezziana, Chrysomya hominivorax, Chrysomya macellaria, Chrysops discalis, Chrysops silacea, Chrysops atlanticus, Cochliomyia hominivorax, Cordylobia anthropophaga, Culicoides furens, Culex pipiens, Culex nigripalpus, Culex quinquefasciatus, Culex tarsalis, Culiseta inornata, Culiseta melanura, Dermatobia hominis, Fannia canicularis, Gasterophilus intestinalis, Glossina morsitans, Glossina palpalis, Glossina fuscipes, Glossina tachinoides, Haematobia irritans, Haplodiplosis equestris, Hippelates spp., Hypoderma lineata, Leptoconops torrens, Lucilia caprina, Lucilia cuprina, Lucilia sericata, Lycoria pectoralis, Mansonia spp., Musca domestica, Muscina stabulans, Oestrus ovis, Phlebotomus argentipes, Psorophora columbiae, Psorophora discolor, Prosimulium mixtum, Sarcophaga haemorrhoidalis, Sarcophaga sp., Simulium vittatum, Stomoxys calcitrans, Tabanus bovinus, Tabanus atratus, Tabanus lineola,* and *Tabanus similis,*
Earwigs *(Dermaptera),* e.g. *forficula auricularia,*
true bugs (Hemiptera), e.g. *Cimex lectularius, Cimex hemipterus, Reduvius senilis, Triatoma* spp., *Rhodnius prolixus,* and *Arilus critatus,*
ants, bees, wasps, sawflies (Hymenoptera), e.g. *Crematogaster spp., Hoplocampa minuta, Hoplocampa testudinea, Monomorium pharaonis, Solenopsis geminata, Solenopsis invicta, Solenopsis richteri, Solenopsis xyloni, Pogonomyrmex barbatus, Pogonomyrmex californicus, Dasymutilla occidentalis, Bombus* spp. *Vespula squamosa, Paravespula vulgaris, Paravespula pennsylvanica, Paravespula germanica, Dolichovespula maculata, Vespa crabro, Polistes rubiginosa, Camponotus floridanus,* and *Linepithema humile,*
crickets, grasshoppers, locusts (Orthoptera), e.g. *Acheta domestica, Gryllotalpa gryllotalpa, Locusta migratoria, Melanoplus bivittatus, Melanoplus femurrubrum, Melanoplus mexicanus, Melanoplus sanguinipes, Melanoplus spretus, Nomadacris septemfasciata, Schistocerca americana, Schistocerca gregaria, Dociostaurus maroccanus, Tachycines asynamorus, Oedaleus senegalensis, Zonozerus variegatus, Hieroglyphus daganensis, Kraussaria angulifera, Calliptamus italicus, Chortoicetes terminifera,* and *Locustana pardalina*,
fleas (Siphonaptera), e.g. *Ctenocephalides felis, Ctenocephalides canis, Xenopsylla cheopis, Pulex irritans, Tunga penetrans,* and *Nosopsyllus fasciatus,*
silverfish, firebrat (Thysanura), e.g. *Lepisma saccharina* and *Thermobia domestica,*
lice (Phthiraptera), e.g. *Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus* and *Solenopotes capillatus.*

The hydrazine derivatives of formula I which can be used according to the invention are known from and can be prepared according to preparation methods described or referenced in EP-A 604 798. This document relates to plant protection in the agricultural field and discloses the insecticidal and acaricidal activity of compounds of fomula I and other compounds against crop pests of the Coleoptera, Lepidoptera and Acarina orders.

In J. A Furch et al., "Amidrazones: A New Class of Coleopteran Insecticides", ACS Symposium Series 686, Am. Chem. Soc., 1998, Chapter 18, p. 178 ff, the use of certain amidrazones against southern corn rootworm is exemplified, some of which amidrazones are compounds of formula I. It is taught that these compounds target specifically certain coleopteran insects while being little toxic or non-toxic to Lepidoptera and other insects as well as to Acarina.

In D. G. Kuhn et al., "Cycloalkyl-substituted Amidrazones: A Novel Class of Insect Control Agents", ACS Symposium Series 686, Am. Chem. Soc., 1998, Chapter 19, p. 185 ff, it is taught that certain amidrazones, some of which amidrazones are compounds of formula I wherein R² and R³ together form a cyclopropyl ring, are active against certain Coleopteran and Lepidoptera species. Biological tests show that the compounds are quite selective, only some exhibiting good activity against both Coleoptera or Lepidoptera.

Activity of a compound against pests for plant protection in the agricultural field, that is, against crop pests, does not generally suggest activity of that compound against non-crop pests. Crop pest control always is a part of plant protection. Non-crop pest control, on the contrary, e.g. relates to protection of non-living organic materials, or hygiene and disease prevention (public health).

The differences in requirements for crop / non-crop pest control generally and mainly - beside a possible difference in biochemical targets - emerge from the differences in the food and / or habitat of the pests.

Crop pests like that of the order Homoptera feed on the green parts of the plant by piercing them and sucking the plant liquids. Other crop pests of the Lepidoptera and Coleoptera order feed on the green parts of plants by biting off parts. On the contrary, non-crop pests do not live on plants and do not or only in rare occasions feed on the green parts of the plant. Non-crop pests e.g. feed on non-living organic materials such as the homes, clothing and the food etc of human beings and animals but also on electric wires etc thereby introducing pathogenic germs into the human being's environment and destroying their homes and food. An example is the termite (order Isoptera) that primarily feeds on cellulose which is the major component of wood and paper products. Another example is the mosquito (order Diptera) whose larvae feed on microorganisms and organic matter in the water and whose adults feed on blood.

The properties of pesticides need to be adapted to their specific use. Systemic pesticides for example that by virtue of their water-solubility are introduced into the plant parts are suitable for controlling piercing-sucking or biting (i.e. crop) pests. However, they cannot generally be expected to show equal activity against non-crop pests who do not feed on the green plant parts but are controlled by mostly water-insoluble pesticides in baiting systems or by direct treatment. In many cases crop pest control pesticides are not suitable for non-crop pest controlling and vice versa. The market insecticides pirimicarb, acephate, pyrimidiven, and pyridaben are examples. They are active against crop insects but show low activity against non-crop pests.

Summing up, prior art teaches the artisan that amidrazones of formula I and of similar structure exhibit selective activity against certain crop pests of the orders Coleoptera or Lepidoptera and that small changes of the structure have a tremendous effect on selectivity.

Suprisingly, it has now been found that a certain group of amidrazones, namely the compounds of formula I, exhibit broad spectrum activity against non-crop pests.

The compounds of formula I and compositions containing them are especially useful for the control of pests of the classes Chilopoda and Diplopoda.

The compounds of formula I and compositions containing them are particularly useful for the control of pests from the orders Isoptera, Blattaria (Blattodea), Diptera, Dermaptera, Hemiptera, Hymenoptera, Orthoptera, Siphonaptera, Thysanura, and Phthiraptera.

Furthermore, the compounds of formula I and compositions containing them are especially useful for the control of pests from the orders Parasitiformes, Araneida and Acaridida.

Moreover, the compounds of formula I and compositions containing them are especially useful for the control of pests from the orders Isoptera, Blattaria (Blattodea), Diptera, Hymenoptera, Siphonaptera, Orthoptera and Ixodida.

More particularly, the compounds of formula I and compositions containing them are especially useful for the control of pests from the orders Isoptera, Blattaria (Blattodea), Diptera, Hymenoptera and Siphonaptera.

In particular, the compounds of formula I and compositions containing them are useful for the control of Isoptera *(Hodotermitidae, Kalotermitidae, Rhinotermitidae, Termitidae),* Blattaria/Blattodea *(Cryptocercidae, Blattidae, Polyphagidae, Blattellidae),* Diptera *(Culicidae, Simuliidae, Ceratopogonidae, Tabanidae, Muscidae, Calliphoridae, Oestridae, Sarcophagidae, Hippoboscidae),* Hymenoptera *(Xyelidae, Argidae, Cimbicidae, Tenthredinidae, Anaxyelidae, Cephidae, Aphidiidae, Formicidae, Vespoidae, Sphecidae),* and Siphonaptera *(Pulicidae, Rhopalopsyllidae, Ceratophyllidae).*

Moreover, the use of the compounds of formula I and compositions containing them for combating pests of the Isoptera order is especially preferred.

The use of the compounds of formula I and compositions containing them for combating pests of the Blattaria/Blattodea order is a further preferred embodiment of the present invention.

Furthermore, the use of the compounds of formula I and compositions containing them for combating pests of the Hymenoptera order, especially ants, is especially preferred.

The use of the compounds of formula I and compositions containing them for combating pests of the Diptera order, especially flies and mosquitoes, is a further preferred embodiment of the present invention.

Furthermore, the use of the compounds of formula I and compositions containing them for combating pests of the Orthoptera order, is especially preferred.

Moreover, the use of the compounds of formula I and compositions containing them for combating pests of the Siphonaptera is especially preferred.

The use of the compounds of formula I and compositions containing them for combating pests of the Ixodida order, especially flies and mosquitoes, is a further preferred embodiment of the present invention.

Furthermore, the use of the compounds of formula I and compositions containing them for combating pests of the Mesostigmata order is a further preferred embodiment of the present invention.

Besides, the use of the compounds of formula I and compositions containing them for combating pests of the Acaridida order is a further preferred embodiment of the present invention.

In a preferred embodiment of the present invention, compounds of formula I and compositions containing them are used for the protection of non-living organic materials, including but are not limited to house-hold goods such as fats, oils, mono- oligo- or polyorganosaccharides, proteins, or fresh or decaying fruits; cellulose-containing materials e.g. wooden materials such as houses, trees, board fences, or sleepers and also paper; and also construction materials, furniture, leathers, animal, plant and synthetic fibers, vinyl articles, electric wires and cables as well as styrene foams.

More preferably, compounds of formula I and compositions containing them are used for the protection of non-living organic materials against non-crop pests selected from the group consisting of the class Diplopoda and of the orders Isoptera, Diptera, Blattaria (Blattodea), Dermaptera, Hemiptera, Hymenoptera, Orthoptera, and Thysanura.

The present invention also relates to a method for the protection of non-living organic materials against non-crop pests, preferably against non-crop pests selected from the group consisting of the class Diplopoda and of the orders Isoptera, Diptera, Blattaria (Blattodea), Dermaptera, Hemiptera, Hymenoptera, Orthoptera, and Thysanura, comprising contacting the pests or their food supply, habitat, breeding grounds, their locus or the non-living organic materials themselves with a pesticidally effective amount of a compound of formula I or a composition containing it.

Moreover, compounds of formula 1 or compositions containing them are preferably used for protecting cellulose-containing non-living organic materials.

Preferably, compounds of formula 1 or compositions containing them are used for protecting cellulose-containing non-living organic materials against non-crop pests from the Isoptera, Diptera, Blattaria (Blattodea), Hymenoptera, and Orthoptera orders, most preferably the Isoptera orders.

The present invention also provides a method for protecting cellulose-containing non-living organic materials against non-crop pests, preferably from the Isoptera, Diptera, Blattaria (Blattodea), Hymenoptera, and Orthoptera orders, most preferably the Isoptera orders, comprising contacting the pests or their food supply, habitat, breeding grounds, their locus or the cellulose-containing non-living organic materials themselves with a pesticidally effective amount of a compound of formula I or a composition containing it.

In another preferred embodiment of the present invention, compounds of formula 1 or compositions comprising them are used for protecting mono- oligo- or polysaccharides and proteins.

Preferably, compounds of formula 1 or compositions comprising them are used for protecting mono- oligo- or polysaccharides and proteins against non-crop pests selected from the Dermaptera, Diplopoda, Isoptera, Diptera, Blattaria (Blattodea), Hymenoptera, Orthoptera and Tysanura orders, most preferably the Isoptera, Diptera, Blattaria (Blattodea), and Hymenoptra orders.

The present invention also provides a method for protecting mono- oligo- or polysaccharides and proteins against non-crop pests, preferably selected from the Dermaptera, Diplopoda, Isoptera, Diptera, Blattaria (Blattodea), Hymenoptera, Orthoptera and Tysanura orders, most preferably the Isoptera, Diptera, Blattaria (Blattodea), and Hymenoptra orders, comprising contacting the pests or their food supply, habitat, breeding grounds or their locus with a pesticidally effective amount of a compound of formula I or a composition containing it.

Furthermore, compounds of formula I are preferably used for protection of animals against non-crop pest of the class Chilopoda, and of the orders Araneida, Hemiptera, Diptera, Phthiraptera, Siphonaptera, Parasitiformes and Acaridida by treatment of the pests in water bodies and/ or in and around buildings, including but not limited to walls, ground, manure piles, turf grass, pastures, sewers and materials used in the construction of buildings and also mattresses and bedding, with a pesticidally effective amount of a compound of formula I or a composition containing it. Most preferably, compounds of formula I are used for protection of animals against non-crop pest of the Diptera, Phthiraptera, Siphonaptera, and Parasitiformes orders.

Animals include warm-blooded animals, including humans and fish. Compounds of formula I are preferably used for protection of warm-blooded animals such as cattle, sheep, swine, camels, deer, horses, poultry, rabbits, goats, dogs and cats.

In the definition of formula I shown above, the substituents have the following meanings:

"Halogen" will be taken to mean fluoro, chloro, bromo and iodo.

The term "alkyl" as used herein refers to a branched or unbranched saturated hydrocarbon group having 1 to 4 or 6 carbon atoms, especially C₁-C₆-alkyl such as methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl.

"Alkoxy" refers to a straight-chain or branched alkyl group having 1 to 4 carbon atoms (methyl, ethyl, propyl, 1-methylethyl, butyl, 1-methylpropyl, 2-methylpropyl, 1,1-dimethylethyl) bonded through an oxygen linkage, at any bond in the alkyl group. Examples include methoxy, ethoxy, propoxy, and isopropoxy.

"Cycloalkyl" refers to a monocyclic 3- to 6-membered saturated carbon atom ring, i.e. cyclopropyl, cyclobutyl, cyclopentyl, and cyclohexyl.

With respect to the intended use of the compounds of formula I, particular preference is given to the following meanings of the substituents, in each case on their own or in combination:

Preference is given to compounds of formula I wherein W is trifluoromethyl.

Preference is further given to compounds of formula I wherein X and Y are both chlorine.

Moreover, preferred are compounds of formula I wherein R¹ is C₁-C₆-alkyl, especially ethyl.

Preference is further given to compounds of formula I wherein R² and R³ are both methyl.

Moreover, preferred are compounds of formula I wherein R² and R³ form a cyclopropyl ring which is unsubstituted or substituted by 1 to 3 halogen atoms, especially chlorine and bromine.

Moreover, particularly preferred are compounds of formula I wherein R² and R³ form a cyclopropyl ring which is substituted by 2 halogen atoms.

Moreover, particularly preferred are compounds of formula I wherein R² and R³ form a cyclopropyl ring which is substituted by 2 chlorine atoms.

Particularly preferred are compounds of formula I wherein R² and R³ form a 2,2-dichlorocyclopropyl ring.

Preference is further given to compounds of formula I wherein R⁴ is C₁-C₆ alkyl, especially methyl.

Particularly preferred are compounds of formula I wherein R², R³ and R⁴ are all methyl.

Moreover, particularly preferred are compounds of formula I wherein R², R³ and R⁴ form a moiety 1-methyl-2,2-dichlorocyclopropyl.

Preference is further given to compounds of formula I wherein
W is trifluoromethyl;
X and Y are each independently chlorine or bromine;
R¹ is C₁-C₆-alkyl;
R² and R³ are C₁-C₆-alkyl or may be taken together to form C₃-C₆-cycloalkyl which is substituted by 1 to 2 halogen atoms;
R⁴ is C₁-C₆-alkyl;
or the enantiomers or salts thereof.

Particular preference is given to N-ethyl-2,2-dimethylpropionamide-2-(2,6-dichloro-α,α,α-trifluoro-p-tolyl)hydrazone and N-Ethyl-2,2-dichloro-1-methylcyclopropane-carboxamide, 2-(2,6-dichloro-α,α,α-trifluoro-p-tolyl)hydrazone.

Furthermore, particular preference with respect to the use in the present invention is given to the compound of formula I-1 (N-ethyl-2,2-dimethylpropionamide-2-(2,6-dichloro-α,α,α-trifluoro-p-tolyl)-hydrazone):

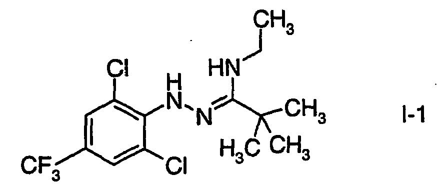

Moreover, particular preference with respect to the use in the present invention is given to the compound of formula I-2 (N-Ethyl-2,2-dichloro-1-methylcyclopropanecarboxamide-2-(2,6-dichloro-α,α,α-tri-fluoro-p-tolyl)hydrazone):

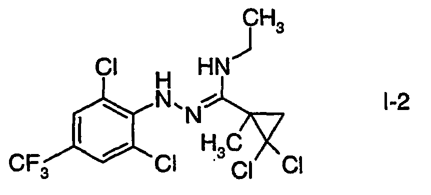

With respect to their use, particular preference is given to the compounds I-A compiled in the tables below. Moreover, the groups mentioned for a substituent in the tables are on their own, independently of the combination in which they are mentioned, a particularly preferred embodiment of the substituent in question.

With respect to their use, particular preference is also given to the hydrochloric acid, maleic acid, dimaleic acid, fumaric acid, difumaric acid, methane sulfenic acid, methane sulfonic acid, and succinic acid adducts of the compounds of the tables below.

Some of the compounds of formula I are new. These are also subject-matter of this invention.

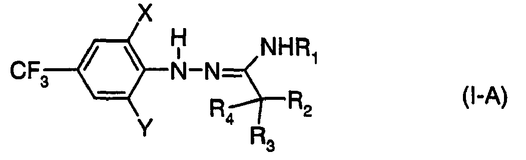

**Table A**

| **No.** | **R¹** | **R²** | **R³** | **R⁴** | **X** | **Y** |
|---|---|---|---|---|---|---|
| A-1 | CH₃ | 2,2-dichlorocyclopropyl | | H | Cl | Cl |
| A-2 | CH₃ | 2,2-dibromocyclopropyl | | H | Cl | Cl |
| A-3 | CH₃ | CH₃ | CH₃ | CH₃ | Cl | Cl |
| A-4 | CH₃ | CH₂CH₃ | CH₃ | CH₃ | Cl | Cl |
| A-5 | CH₃ | 2,2-dichlorocyclopropyl | | CH₃ | Cl | Cl |
| A-6 | CH₃ | 2,2-dibromocyclopropyl | | CH₃ | Cl | Cl |
| A-7 | CH₃ | 2,2-dichlorocyclopropyl | | H | Br | Br |
| A-8 | CH₃ | 2,2-dibromocyclopropyl | | H | Br | Br |
| A-9 | CH₃ | CH₃ | CH₃ | CH₃ | Br | Br |
| A-10 | CH₃ | CH₂CH₃ | CH₃ | CH₃ | Br | Br |
| A-11 | CH₃ | 2,2-dichlorocyclopropyl | | CH₃ | Br | Br |
| A-12 | CH₃ | 2,2-dibromocyclopropyl | | CH₃ | Br | Br |
| A-13 | CH₂CH₃ | 2,2-dichlorocyclopropyl | | H | Cl | Cl |
| A-14 | CH₂CH₃ | 2,2-dibromocyclopropyl | | H | Cl | Cl |
| A-15 | CH₂CH₃ | CH₃ | CH₃ | CH₃ | Cl | Cl |
| A-16 | CH₂CH₃ | CH₂CH₃ | CH₃ | CH₃ | Cl | Cl |
| A-17 | CH₂CH₃ | 2,2-dichlorocyclopropyl | | CH₃ | Cl | Cl |
| A-18 | CH₂CH₃ | 2,2-dibromocyclopropyl | | CH₃ | Cl | Cl |
| A-19 | CH₂CH₃ | 2,2-dichlorocyclopropyl | | H | Br | Br |
| A-20 | CH₂CH₃ | 2,2-dibromocyclopropyl | | H | Br | Br |
| A-21 | CH₂CH₃ | CH₃ | CH₃ | CH₃ | Br | Br |
| A-22 | CH₂CH₃ | CH₂CH₃ | CH₃ | CH₃ | Br | Br |
| A-23 | CH₂CH₃ | 2,2-dichlorocyclopropyl | | CH₃ | Br | Br |
| A-24 | CH₂CH₃ | 2,2-dibromocyclopropyl | | CH₃ | Br | Br |
| A-25 | CH₂CH₂CH₃ | 2,2-dichlorocyclopropyl | | H | Cl | Cl |
| A-26 | CH₂CH₂CH₃ | 2,2-dibromocyclopropyl | | H | Cl | Cl |
| A-27 | CH₂CH₂CH₃ | CH₃ | CH₃ | CH₃ | Cl | Cl |
| A-28 | CH₂CH₂CH₃ | CH₂CH₃ | CH₃ | CH₃ | Cl | Cl |
| A-29 | CH₂CH₂CH₃ | 2,2-dichlorocyclopropyl | | CH₃ | Cl | Cl |
| A-30 | CH₂CH₂CH₃ | 2,2-dibromocyclopropyl | | CH₃ | Cl | Cl |
| A-31 | CH₂CH₂CH₃ | 2,2-dichlorocyclopropyl | | H | Br | Br |
| A-32 | CH₂CH₂CH₃ | 2,2-dibromocyclopropyl | | H | Br | Br |
| A-33 | CH₂CH₂CH₃ | CH₃ | CH₃ | CH₃ | Br | Br |
| A-34 | CH₂CH₂CH₃ | CH₂CH₃ | CH₃ | CH₃ | Br | Br |
| A-35 | CH₂CH₂CH₃ | 2,2-dichlorocyclopropyl | | CH₃ | Br | Br |
| A-36 | CH₂CH₂CH₃ | 2,2-dibromocyclopropyl | | CH₃ | Br | Br |
| A-37 | C₃H₅ | 2,2-dichlorocyclopropyl | | H | Cl | Cl |
| A-38 | C₃H₅ | 2,2-dibromocyclopropyl | | H | Cl | Cl |
| A-39 | C₃H₅ | CH₃ | CH₃ | CH₃ | Cl | Cl |
| A-40 | C₃H₅ | CH₂CH₃ | CH₃ | CH₃ | Cl | Cl |
| A-41 | C₃H₅ | 2,2-dichlorocyclopropyl | | CH₃ | Cl | Cl |
| A-42 | C₃H₅ | 2,2-dibromocyclopropyl | | CH₃ | Cl | Cl |
| A-43 | C₃H₅ | 2,2-dichlorocyclopropyl | | H | Br | Br |
| A-44 | C₃H₅ | 2,2-dibromocyclopropyl | | H | Br | Br |
| A-45 | C₃H₅ | CH₃ | CH₃ | CH₃ | Br | Br |
| A-46 | C₃H₅ | CH₂CH₃ | CH₃ | CH₃ | Br | Br |
| A-47 | C₃H₅ | 2,2-dichlorocyclopropyl | | CH₃ | Br | Br |
| A-48 | C₃H₅ | 2,2-dibromocyclopropyl | | CH₃ | Br | Br |
| A-49 | CH₂OCH₃ | 2,2-dichlorocyclopropyl | | H | Cl | Cl |
| A-50 | CH₂OCH₃ | 2,2-dibromocyclopropyl | | H | Cl | Cl |
| A-51 | CH₂OCH₃ | CH₃ | CH₃ | CH₃ | Cl | Cl |
| A-52 | CH₂OCH₃ | CH₂CH₃ | CH₃ | CH₃ | Cl | Cl |
| A-53 | CH₂OCH₃ | 2,2-dichlorocyclopropyl | | CH₃ | Cl | Cl |
| A-54 | CH₂OCH₃ | 2,2-dibromocyclopropyl | | CH₃ | Cl | Cl |
| A-55 | CH₂OCH₃ | 2,2-dichlorocyclopropyl | | H | Br | Br |
| A-56 | CH₂OCH₃ | 2,2-dibromocyclopropyl | | H | Br | Br |
| A-57 | CH₂OCH₃ | CH₃ | CH₃ | CH₃ | Br | Br |
| A-58 | CH₂OCH₃ | CH₂CH₃ | CH₃ | CH₃ | Br | Br |
| A-59 | CH₂OCH₃ | 2,2-dichlorocyclopropyl | | CH₃ | Br | Br |
| A-60 | CH₂OCH₃ | 2,2-dibromocyclopropyl | | CH₃ | Br | Br |
| A-61 | CH₂OCH₂CH₃ | 2,2-dichlorocyclopropyl | | H | Cl | Cl |
| A-62 | CH₂OCH₂CH₃ | 2,2-dibromocyclopropyl | | H | Cl | Cl |
| A-63 | CH₂OCH₂CH₃ | CH₃ | CH₃ | CH₃ | Cl | Cl |
| A-64 | CH₂OCH₂CH₃ | CH₂CH₃ | CH₃ | CH₃ | Cl | Cl |
| A-65 | CH₂OCH₂CH₃ | 2,2-dichlorocyclopropyl | | CH₃ | Cl | Cl |
| A-66 | CH₂OCH₂CH₃ | 2,2-dibromocyclopropyl | | CH₃ | Cl | Cl |
| A-67 | CH₂OCH₂CH₃ | 2,2-dichlorocyclopropyl | | H | Br | Br |
| A-68 | CH₂OCH₂CH₃ | 2,2-dibromocyclopropyl | | H | Br | Br |
| A-69 | CH₂OCH₂CH₃ | CH₃ | CH₃ | CH₃ | Br | Br |
| A-70 | CH₂OCH₂CH₃ | CH₂CH₃ | CH₃ | CH₃ | Br | Br |
| A-71 | CH₂OCH₂CH₃ | 2,2-dichlorocyclopropyl | | CH₃ | Br | Br |
| A-72 | CH₂OCH₂CH₃ | 2,2-dibromocyclopropyl | | CH₃ | Br | Br |

For use according to the present invention, the compounds I can be converted into the customary formulations, e.g. solutions, emulsions, suspensions, dusts, powders, pastes and granules. The use form depends on the particular purpose; it is intended to ensure in each case a fine and uniform distribution of the compound according to the invention.

The formulations are prepared in a known manner, e.g. by extending the active ingredient with solvents and/or carriers, if desired using emulsifiers and dispersants. Solvents/auxiliaries, which are suitable, are essentially:
- water, aromatic solvents (for example Solvesso products, xylene), paraffins (for example mineral fractions), alcohols (for example methanol, butanol, pentanol, benzyl alcohol), ketones (for example cyclohexanone, gamma-butyrolactone), pyrrolidones (NMP, NOP), acetates (glycol diacetate), glycols, fatty acid dimethylamides, fatty acids and fatty acid esters. In principle, solvent mixtures may also be used.
- carriers such as ground natural minerals (e.g. kaolins, clays, talc, chalk) and ground synthetic minerals (e.g. highly disperse silica, silicates); emulsifiers such as nonionic and anionic emulsifiers (e.g. polyoxyethylene fatty alcohol ethers, alkylsulfonates and arylsulfonates) and dispersants such as lignin-sulfite waste liquors and methylcellulose.

Suitable surfactants are alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol ®octylphenol, nonylphenol, alkylphenyl polyglycol ethers, tributylphenyl polyglycol ether, tristearylphenyl polyglycol ether, alkylaryl polyether alcohols, alcohol and fatty alcohol/ethylene oxide condensates, ethoxylated castor oil, polyoxyethylene alkyl ethers, ethoxylated polyoxypropylene, lauryl alcohol polyglycol ether acetal, sorbitol esters, lignin-sulfite waste liquors and methylcellulose and ethylene oxide / propylene oxide block copolymers.

Substances which are suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin; tetrahydronaphthalene, alkylated naphthalenes or their derivatives, methanol, ethanol, propanol, butanol, cyclohexanol, cyclohexanone, isophorone, strongly polar solvents, for example dimethyl sulfoxide, N-methylpyrrolidone and water.

Powders, materials for spreading and dusts can be prepared by mixing or concomitantly grinding the active substances with a solid carrier.

Granules, for example coated granules, impregnated granules and homogeneous granules, can be prepared by binding the active ingredients to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nutshell meal, cellulose powders and other solid carriers.

In general, the formulations comprise from 0.01 to 95% by weight, preferably from 0.1 to 90% by weight, of the active ingredient. The active ingredients are employed in a purity of from 90% to 100%, preferably 95% to 100% (according to NMR spectrum).

The following are examples of formulations: 1. Products for dilution with water

### A Soluble concentrates (SL)

10 parts by weight of a compound according to the invention are dissolved in water or in a water-soluble solvent. As an alternative, wetters or other auxiliaries are added. The active ingredient dissolves upon dilution with water.

### B Dispersible concentrates (DC)

20 parts by weight of a compound according to the invention are dissolved in cyclohexanone with addition of a dispersant, for example polyvinylpyrrolidone. Dilution with water gives a dispersion.

### C Emulsifiable concentrates (EC)

15 parts by weight of a compound according to the invention are dissolved in xylene with addition of calcium dodecylbenzenesulfonate and castor oil ethoxylate (in each case 5% strength). Dilution with water gives an emulsion.

### D Emulsions (EW, EO)

40 parts by weight of a compound according to the invention are dissolved in xylene with addition of calcium dodecylbenzenesulfonate and castor oil ethoxylate (in each case 5% strength). This mixture is introduced into water by means of an emulsifier (Ultraturrax) and made into a homogeneous emulsion. Dilution with water gives an emulsion.

### E Suspensions (SC, OD)

In an agitated ball mill, 20 parts by weight of a compound according to the invention are milled with addition of dispersant, wetters and water or an organic solvent to give a fine active ingredient suspension. Dilution with water gives a stable suspension of the active ingredient.

### F Water-dispersible granules and water-soluble granules (WG)

50 parts by weight of a compound according to the invention are ground finely with addition of dispersants and wetters and made into water-dispersible or water-soluble granules by means of technical appliances (for example extrusion, spray tower, fluidized bed). Dilution with water gives a stable dispersion or solution of the active ingredient.

### G Water-dispersible powders and water-soluble powders (WP, SP)

75 parts by weight of a compound according to the invention are ground in a rotor-stator mill with addition of dispersant, wetters and silica gel. Dilution with water gives a stable dispersion or solution with the active ingredient.

### 2. Products to be applied undiluted

### H Dustable powders (DP)

5 parts by weight of a compound according to the invention are ground finely and mixed intimately with 95% of finely divided kaolin. This gives a dustable product.

### I Granules (GR, FG, GG, MG)

0.5 parts by weight of a compound according to the invention is ground finely and associated with 95.5% carriers. Current methods are extrusion, spray drying or the fluidized bed. This gives granules to be applied undiluted.

### J ULV solutions (UL)

10 parts by weight of a compound according to the invention are dissolved in an organic solvent, for example xylene. This gives a product to be applied undiluted.

The active ingredients can be used as such, in the form of their formulations or the use forms prepared therefrom, eg. in the form of directly sprayable solutions, powders, gels, suspensions or dispersions, emulsions, oil dispersions, pastes, dustable products, materials for spreading, or granules, microcapsules (CS), pellets or tablets, by means of spraying, atomizing, dusting, spreading or pouring. The use forms depend entirely on the intended purposes; it is intended to ensure in each case the finest possible distribution of the active ingredients according to the invention.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. Alternatively, it is possible to prepare concentrates composed of active substance, wetter, tackifier, dispersant or emulsifier and, if appropriate, solvent or oil, and such concentrates are suitable for dilution with water.

The active ingredient concentrations in the ready-to-use products can be varied within relatively wide ranges. In general, they are from 0.0001 to 10%, preferably from 0.01 to 1%.

The active ingredients may also be used successfully in the ultra-low-volume process (ULV), it being possible to apply formulations comprising over 95% by weight of active ingredient, or even to apply the active ingredient without additives.

Various, types of oils, wetters, adjuvants, herbicides, fungicides, other pesticides, or bactericides may be added to the active ingredients, if appropriate just immediately prior to use. These agents usually are admixed with the agents according to the invention in a weight ratio of 1:10 to 10:1.

The compounds of formula I are effective through both direct and indirect contact and ingestion, and also through trophallaxis and transfer.

Preferred application methods are into water bodies, via soil, cracks and crevices, pastures, manure piles, sewers, into water, on floor, wall, or by perimeter spray application and bait.

According to a preferred embodiment of the invention, the compounds of formula I are employed via soil application. Soil application is especially favorable for use against ants, termites, wasps, crickets, or cockroaches.

According to another preferred embodiment of the invention, for use against non crop pests such as ants, termites, wasps, flies, mosquitoes, crickets, locusts, cockroaches, and firebrats the compounds of formula I are prepared into a bait preparation.

The bait can be a liquid, a solid or a semisolid preparation (e.g. a gel). Solid baits can be formed into various shapes and forms suitable to the respective application e.g. granules, blocks, sticks, disks. Liquid baits can be filled into various devices to ensure proper application, e.g. open containers, spray devices, droplet sources, or evaporation sources. Gels can be based on aqueous or oily matrices and can be formulated to particular necessities in terms of stickiness, moisture retention or aging characteristics.

The bait employed in the composition is a product which is sufficiently attractive to incite insects such as ants, termites, wasps, flies, mosquitoes, crickets etc. or cockroaches to eat it. This attractant may be chosen from feeding stimulants or para and / or sex pheromones. Suitable feeding stimulants are chosen, for example, from animal and/or plant proteins (meat-, fish- or blood meal, insect parts, crickets powder, egg yolk), from fats and oils of animal and/or plant origin, or mono-, oligo- or polyorganosaccharides, especially from sucrose, lactose, fructose, dextrose, glucose, starch, pectin or even molasses or honey, or from salts such as ammonium sulfate, ammonium carbonate or ammonium acetate. Fresh or decaying parts of fruits, crops, plants, animals, insects or specific parts thereof can also serve as a feeding stimulant.
Pheromones are known to be more insect specific. Specific pheromones are described in the literature and are known to those skilled in the art.

Formulations of compounds of formula I as aerosols (e.g in spray cans), oil sprays or pump sprays are highly suitable for the non-professional user for controlling pests such as flies, fleas, ticks, mosquitoes, locusts or cockroaches. Aerosol recipes are preferably composed of the active compound, solvents such as lower alcohols (e.g. methanol, ethanol, propanol, butanol), ketones (e.g. acetone, methyl ethyl ketone), paraffin hydrocarbons (e.g. kerosenes) having boiling ranges of approximately 50 to 250 °C, dimethylformamide, N-methylpyrrolidone, dimethyl sulphoxide, aromatic hydrocarbons such as toluene, xylene, water, furthermore auxiliaries such as emulsifiers such as sorbitol monooleate, oleyl ethoxylate having 3-7 mol of ethylene oxide, fatty alcohol ethoxylate, perfume oils such as ethereal oils, esters of medium fatty acids with lower alcohols, aromatic carbonyl compounds, if appropriate stabilizers such as sodium benzoate, amphoteric surfactants, lower epoxides, triethyl orthoformate and, if required, propellants such as propane, butane, nitrogen, compressed air, dimethyl ether, carbon dioxide, nitrous oxide, or mixtures of these gases.

The oil spray formulations differ from the aerosol recipes in that no propellants are used.

The compounds of formula I and its respective compositions can also be used in mosquito coils and fumigating coils, smoke cartridges, vaporizer plates, long-term vaporizers, or other heat-independent vaporizer systems.

Methods to control infectious diseases transmitted by insects (e.g. malaria, dengue and yellow fever, lymphatic filariasis, and leishmaniasis) with compounds of formula I and its respective compositions also comprise treating surfaces of huts and houses, air spraying and impregnation of curtains, tents, clothing items, bed nets, tsetse-fly trap or the like. Insecticidal compositions for application to fibers, fabric, knitgoods, nonwovens, netting material or foils and tarpaulins preferably comprise a mixture including the insecticide, optionally a repellent and at least one binder.

The impregnation of curtains and bed nets is mostly done by dipping the textile material into emulsions or dispersions of the insecticide or spraying them onto the nets.

The following list of pesticides together with which the compounds according to the invention can be used, is intended to illustrate the possible combinations, but not to impose any limitation:
Organophosphates: Acephate, Azinphos-methyl, Chlorpyrifos, Chlorfenvinphos, Diazinon, Dichlorvos, Dicrotophos, Dimethoate, Disulfoton, Ethion, Fenitrothion, Fenthion, Isoxathion, Malathion, Methamidophos, Methidathion, Methyl-Parathion, Mevinphos, Monocrotophos, Oxydemeton-methyl, Paraoxon, Parathion, Phenthoate, Phosalone, Phosmet, Phosphamidon, Phorate, Phoxim, Pirimiphos-methyl, Profenofos, Prothiofos, Sulprophos, Terbufos, Triazophos, Trichlorfon;
Carbamates: Alanycarb, Benfuracarb, Carbaryl, Carbosulfan, Fenoxycarb, Furathiocarb, Indoxacarb, Methiocarb, Methomyl, Oxamyl, Pirimicarb, Propoxur, Thiodicarb, Triazamate;
Pyrethroids: Bifenthrin, Cyfluthrin, Cypermethrin, alpha-Cypermethrin, Deltamethrin, Esfenvalerate, Ethofenprox, Fenpropathrin, Fenvalerate, Cyhalothrin, Lambda-Cyhalothrin, Permethrin, Silafluofen, Tau-Fluvalinate, Tefluthrin, Tralomethrin, Zeta-Cypermethrin;
Arthropod growth regulators: a) chitin synthesis inhibitors: benzoylureas: Chlorfluazuron, Diflubenzuron, Flucycloxuron, Flufenoxuron, Hexaflumuron, Lufenuron, Novaluron, Teflubenzuron, Triflumuron; Buprofezin, Diofenolan, Hexythiazox, Etoxazole, Clofentazine; b) ecdysone antagonists: Halofenozide, Methoxyfenozide, Tebufenozide; c) juvenoids: Pyriproxyfen, Methoprene, Fenoxycarb; d) lipid biosynthesis inhibitors: Spirodiclofen;
Neonicotinoids: Acetamiprid, Clothianidin, Flonicamid, Imidacloprid, Nitenpyram, Thiacloprid, Thiamethoxam;

Various: Abamectin, Acequinocyl, Amitraz, Azadirachtin, Bifenazate, *Bacillus thuringiensis, Bacillus subtilis,* Cartap, Chlorfenapyr, Chlordimeform, Cyromazine, Diafenthiuron, Dinetofuran, Diofenolan, Emamectin, Endosulfan, Ethiprole, Fenazaquin, Fipronil, Formetanate, Formetanate hydrochloride, Hydramethylnon, Indoxacarb, 4-{(2Z)-2-({[4-(trifluoro-methoxy)anilino] carbonyl} hydrazono)-2-[3-(trifluoromethyl)-phenyl]ethyll benzo-nitrile, Pyridaben, Pymetrozine, Spinosad, Sulfur, Tebufenpyrad, and Thiocyclam.

The insects may be controlled by contacting the target parasite/pest, its food supply, habitat, breeding ground or its locus with a pesticidally effective amount of compounds of or compositions of formula I.

"Locus" means a habitat, breeding ground, soil, area, material or environment in which a pest or parasite is growing or may grow.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application, and the like.

The compounds of formula I and its compositions can be used for protecting wooden materials such as trees, board fences, sleepers, etc. and buildings such as houses, outhouses, factories, but also construction materials, furniture, leathers, fibers, vinyl articles, electric wires and cables etc. from ants and/or termites, and for controlling ants and termites from doing harm to crops or human being (e.g. when the pests invade into houses and public facilities). The compounds of formula I are applied not only to the surrounding soil surface or into the under-floor soil in order to protect wooden materials but it can also be applied to lumbered articles such as surfaces of the under-floor concrete, alcove posts, beams, plywoods, furniture, etc., wooden articles such as particle boards, half boards, etc. and vinyl articles such as coated electric wires, vinyl sheets, heat insulating material such as styrene foams, etc. In case of application against ants doing harm to crops or human beings, the ant control composition of the present invention is directly applied to the nest of the ants or to its surrounding or via bait contact.

The compounds or compositions of the invention can also be applied preventively to places at which occurrence of the pests is expected.

In the case of soil treatment or of application to the pests dwelling place or nest, the quantity, of active ingredient ranges from 0.0001 to 500 g per 100 m², preferably from 0.001 to 20 g per 100 m². Soil treatment of termites (Isoptera) is an especially preferred embodiment of the present invention..

Customary application rates in the protection of materials are, for example, from 0.01 g to 1000 g of active compound per m² treated material, desirably from 0.1 g to 50 g per m²_{.}

Insecticidal compositions for use in the impregnation of materials typically contain from 0.001 to 95 weight %, preferably from 0.1 to 45 weight %, and more preferably from 1 to 25 weight % of at least one repellent and / or insecticide.

For use in bait compositions, the typical content of active ingredient is from 0.0001 weight % to 15 weight %, desirably from 0.001 weight % to 5% weight % of active compound. The composition used may also comprise other additives such as a solvent of the active material, a flavoring agent, a preserving agent, a dye or a bitter agent. Its attractiveness may also be enhanced by a special colour, shape or texture.

For use in spray compositions, the content of active ingredient is from 0.001 to 80 weights %, preferably from 0.01 to 50 weight % and most preferably from 0.01 to 15 weight %.

### Examples of action against pests

### Test Methodology

### 1. Activity against Argentine ant, harvester ant, acrobat ant, carpenter ant, fire ant, house fly, stable fly, flesh fly, yellowfever mosquito, house mosquito, malaria mosquito, German cockroach, cat flea, and brown dog tick via glass contact

Glass vials (20 ml scintillation vials) were treated with 0.5 ml of a solution of active ingredient in acetone. Each vial was rolled uncapped for ca. 10 minutes to allow the a.i. to completely coat the vial and to allow for full drying of the acetone. Insects or ticks were placed into each vial. The vials were kept at 22 °C and were observed for treatment effects at various time intervals. Results are presented in Table I.

### 2. Activity against Argentine ant, acrobat ant, carpenter ant, fire ant, and eastern subterranean termite via soil contact

For ants, tests were conducted in Petri dishes. A thin layer of 1 % agar in water was dispensed into the dishes and Florida sandy soil was spread over the agar (5 g for the small dishes and 11 g for the larger dishes). The active ingredient was dissolved in acetone and dispensed over the sand. Dishes were vented to evaporate the acetone, infested with ants, and covered. A 20% honey water solution was placed in each dish. The dishes were maintained at 22°C and observed for mortality at various time intervals.

For termites, a thin layer of 1% agar was dispensed into Petri dishes. A thin layer of pre-treated soil was spread over the agar. For soil treatment, the active ingredient was diluted in acetone on a weight-to-weight basis and incorporated into 100 g of soil. The soil was placed in a jar and vented for 48 hours. The moisture level of the soil was brought to field capacity by adding 7 ml of water. Termite workers were introduced into each dish. A small piece of filter paper was placed into each dish after 1 day as a food source, and additional water was added as needed to maintain soil moisture. Test dishes were held at a dark incubator at 25°C and appr. 80% relative humidity. Termites were observed daily for mortality (dead or unable to stand upright and showing only weak movement). Results are shown in Table 1.

### 3. Activity against Argentine ant, acrobat ant, carpenter ant, fire ant, house fly, eastern subterranean termite, formosan subterranean termite, and German cockroach via bait

For Argentine ant, acrobat ant, and carpenter ant, tests were conducted in Petri dishes. Ants were given a water source, and then were starved of a food source for 24 hours. Baits were prepared with either 20% honey/water solutions or ground cat chow. Active ingredient in acetone was added to the bait. 0.2 ml of treated honey water solution or 150 mg of treated cat chow, placed in a cap, was added to each dish. The dishes were covered and maintained at a temperature of 22°C. The ants were observed for mortality daily. Results are shown in Table I.

For the fire ants, corn grit was used as a bait matrix. Corn grit bait was prepared using a mixture of defatted corn grit (80%), soybean oil (19.9%), acetone, and the active ingredient (0.1%). Petri dishes were supplied with a water source. Fire ant adults were placed into each dish. The next day, 250 mg of bait in bait containers was placed into the dishes. The ants were observed for mortality daily. Results are shown in Table I.

For house flies. Bait tests were conducted with adults aged 2-5 days post-emergence. Active ingredient in acetone was applied to a bait matrix consisting of a 1:1 mixture of powdered milk and sugar which was then allowed to dry. Assays were conducted in jars with 250 mg of bait in a pan placed in the bottom of each jar. House flies were placed into the bait jars which were covered. The test jars were held at 22°C. Test jars were observed at 4 hours after treatment for knockdown (death plus morbidity (unable to stay upright). Results are shown in Table I.

For termites, active ingredient in acetone was applied to filter papers. % a.i. were calculated on basis of the weight of the filter paper. Acetone only was applied for untreated controls. Treated papers were vented to evaporate the acetone, moistened with ml water, and placed Petri dishes with sand. Water was added during the test as needed. Bioassays were conducted with one treated filter and ca. 30 termite workers per test dish. Test dishes were maintained at 25°C and appr. 85% relative humidity and observed daily for mortality (dead or moribund insects) or intoxication. Dead or moribund insects were removed daily. Results are shown in Table t.

For cockroaches, plastic roach boxes with ventilated lids were used as test arenas. The top 3-4 cm of the arenas was treated with Vaseline and mineral oil to prevent roaches from escaping. Water was provided as needed. The bait was prepared using ground cat chow, and the active ingredient in acetone was incorporated on a weight- to-weight ratio. The treated chow was allowed to dry. The cockroaches were placed in the boxes and starved for 24 hours prior to bait introduction. 0.03 grams of bait per box were placed in a weigh boat. The boxes were maintained at 22°C and observed daily for mortality of the cockroaches. Results are shown in Table 1.

### 4. Activity against yellowfever mosquito, southern house mosquito, and malaria mosquito larvae via water treatment

Well plates were used as test arenas. The active ingredient was dissolved in acetone and diluted with water to obtain the concentrations needed. The final solutions containing appr. 1% acetone were placed into each well. Approximately 10 mosquito larvae (4^{th}-instars) in 1 ml water were added to each well. Larvae were fed one drop of liver powder each day. The dishes were covered and maintained at 22°C. Mortality was recorded daily and dead larvae and live or dead pupae were removed daily. At the end of the test remaining live larvae were recorded and percent mortality was calculated. Results are shown in Table I.

Each test was replicated at least 3 times.

### Results

Tests conducted with compounds of formula I-1 and I-2 showed the following results:

**Table I. Activity against various species.**

| Pest Common Name | Pest Latin Name | Rate | Days or Hours to achieve 100% mortality |
|---|---|---|---|
| Activity via glass contact | | | |
| Argentine ant | *Linepithema humile* | 10 ppm | 1-2 days |
| harvester ant | *Pogonomyrmex californicus* | 10 ppm | 2-3 days |
| acrobat ant | *Crematogaster spp.* | 10 ppm | 1-2 days |
| carpenter ant | *Camponotus floridanus* | 10 ppm | 1 day |
| fire ant | *Solenopsis invicta* | 10 ppm | 4 hours |
| house fly | *Musca domestica* | 10 ppm | 4 hours |
| stable fly | *Stomoxys calcitrans* | 10 ppm | 4 hours |
| flesh fly | *Sarcophaga sp.* | 10 ppm | 4 hours |
| yellowfever mosquito | *Aedes aegypti* | 10 ppm | 4 hours |
| house mosquito | *Culex quinquefasciatus* | 0.5 ppm | 4 hours |
| malaria mosquito | *Anopheles albimanus* | 1 ppm | 1 day |
| German cockroach | *Blattella germanica* | 100 ppm | 5-24 hours |
| cat flea | *Ctenocephalides felis* | 100 ppm | 2 days |
| brown dog tick | *Rhipicephalus sanguineus* | 10 ppm | 3-5 days |

| Activity via soil contact | | | |
|---|---|---|---|
| Argentine ant | *Linepithema humile* | 0.1w% | 1-2 days |
| acrobat ant | *Crematogaster spp.* | 0.01w% | 2 days |
| carpenter ant | *Camponotus floridanus* | 0.01w% | 1 day |
| fire ant | *Solenopsis invicta* | 0.01w% | 1 day |
| subterranean termite | *Reticulitermes flavipes* | 0.005w% | 1-3 days |

| Activity via bait | | | |
|---|---|---|---|
| Argentine ant | *Linepithema humile* | 1.0w% | 2-3 days |
| acrobat ant | *Crematogaster spp.* | 1.5w% | 6-7 days |
| carpenter ant | *Camponotus floridanus* | 2.0w% | 1-3 days |
| fire ant | *Solenopsis invicta* | 0.7w% | 3 days |
| house fly | *Musca domestica* | 0.1w% | 3 hours |
| subterranean termite | *Reticulitermes flavipes* | 0.1w% | 1-2 days |
| Formosan termite | *Coptotermes formosanus* | 0.1w% | 5 days |
| German cockroach | *Blattella germanica* | 0.3w% | 1-2 days |

| Activity via water treatment | | | |
|---|---|---|---|
| yellowfever mosquito | *Aedes aegypti* | 10 ppm | 2 days |
| house mosquito | *Culex quinquefasciatus* | 10 ppm | 1 day |
| malaria mosquito | *Anopheles albimanus* | 1.0 ppm | 1 day |

## Claims

1. The use of compounds of formula I 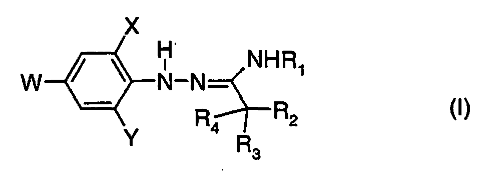 wherein
W is chlorine or trifluoromethyl;
X and Y are each independently chlorine or bromine;
R¹ is C₁-C₆-alkyl, C₃-C₆-alkenyl, C₃-C₆-alkynyl, or C₃-C₆-cycloalkyl which may be substituted with 1 to 3 halogen atoms, or C₂-C₄-alkyl which is substituted by C₁-C₄-alkoxy;
R² and R³ are C₁-C₆-alkyl or may be taken together to form C₃-C₆-cycloalkyl which may be unsubstituted or substituted by 1 to 3 halogen atoms;
R⁴ is hydrogen or C₁-C₆-alkyl,
or the enantiomers or salts thereof,
for combating non-crop pests.

2. The use according to claim 1 wherein the non-crop pests are selected from the group consisting of the classes Chilopoda and Diplopoda and of the orders Isoptera, Diptera, Blattaria (Blattodea), Dermaptera, Hemiptera, Hymenoptera, Orthoptera, Siphonaptera, Thysanura, Phthiraptera, Araneida, Parasitiformes and Acaridida.

3. The use according to claims 1 or 2 wherein the non-crop pests are selected from the group consisting of the orders Isoptera, Blattaria (Blattodea), Diptera, Hymenoptera, Siphonaptera, Orthoptera, and lxodida.

4. The use of the compounds of formula I as defined in claim 1 for the protection of non-living organic materials.

5. The use according to claim 4 for the protection of non-living organic materials against non-crop pests selected from the group consisting of the class Diplopoda and of the orders Isoptera, Diptera, Blattaria (Blattodea), Dermaptera, Hemiptera, Hymenoptera, Orthoptera, and Thysanura.

6. The use according to claims 1 to 5 wherein the compound of formula I is a compound of formula I-1.

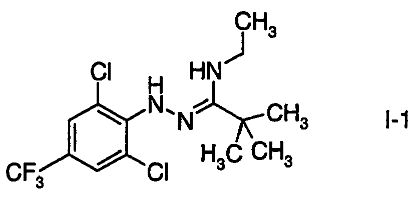

7. The use according to claims 1 to 5 wherein the compound of formula I is a compound of formula I-2.

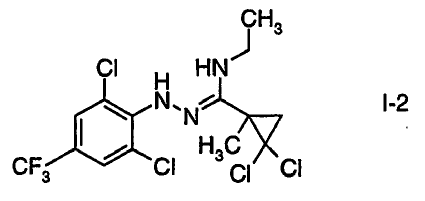

8. A method for controlling non-crop pests comprising contacting the pests or their food supply, habitat, breeding grounds or their locus with a pesticidally effective amount of a compound of formula I as defined in claims 1, 6 or 7.

9. A method according to claim 8 wherein the non-crop pests are selected from the group consisting of the classes Chilopoda and Diplopoda and of the orders Isoptera, Diptera, Blattaria (Blattodea), Dermaptera, Hemiptera, Hymenoptera, Orthoptera, Siphonaptera, Thysanura, Phthiraptera, Araneida, Parasitiformes and Acaridida.

10. A method according to claims 8 or 9 wherein the non-crop pests are selected from the group consisting of the orders Isoptera, Blattaria (Blattodea), Diptera, Hymenoptera, Siphonaptera, Orthoptera, and Ixodida.

11. A method for the protection of non-living organic materials against non-crop pests selected from the group consisting of the class Diplopoda and of the orders Isoptera, Diptera, Blattaria (Blattodea), Dermaptera, Hemiptera, Hymenoptera, Orthoptera, and Thysanura comprising contacting the pests or their food supply, habitat, breeding grounds, their locus or the non-living organic materials with a pesticidally effective amount of a compound of formula I as defined in claims 1, 6 or 7.

12. A method for the protection of animals against non-crop pests selected from the group consisting of the class Chilopoda and of the orders Araneida, Hemiptera, Diptera, Phthiraptera, Siphonaptera, Parasitiformes and Acaridida, comprising treatment of the pests in water bodies and/or in and around buildings with a pesticidally effective amount of a compound of formula I as defined in claims 1, 6 or 7.

13. A method according to claim 12 wherein the non-crop pests are selected from the group consisting of the Diptera, Phthiraptera, Siphonaptera, and Parasitiformes orders.

14. A bait composition which comprises a pesticidally effective amount of a compound of formula I as defined in claims 1, 6 or 7 and an attractant.

## Patentansprüche

1. Verwendung von Verbindungen der Formel I 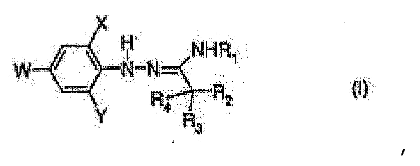 in der
W Chlor oder Trifluormethyl bedeutet;
X und Y jeweils unabhängig Chlor oder Brom bedeuten;
R¹ C₁-C₆-Alkyl, C₃-C₆-Alkenyl, C₃-C₆-Alkinyl, oder C₃-C₆-Cycloalkyl, das durch 1 bis 3 Halogenatome substituiert sein kann, oder C₂-C₄-Alkyl, das durch C₁-C₄-Alkoxy substituiert ist, bedeutet;
R² und R³ C₁-C₆-Alkyl bedeuten oder gemeinsam C₃-C₆-Cycloalkyl, das unsubstituiert oder durch 1 bis 3 Halogenatome substituiert sein kann, bilden können;
R⁴ Wasserstoff oder C₁-C₆-Alkyl bedeutet,
oder von ihren Enantiomeren oder Salzen
zur Bekämpfung von Nichtkulturpflanzenschädlingen.

2. Verfahren nach Anspruch 1, wobei die Nichtkulturpflanzenschädlinge aus der Gruppe bestehend aus den Klassen Chilopoda und Diplopoda und aus den Ordnungen Isoptera, Diptera, Blattaria (Blattodea), Dermaptera, Hemiptera, Hymenoptera, Orthoptera, Siphonaptera, Thysanura, Phthiraptera, Araneida, Parasitiformes und Acaridida stammen.

3. Verbindung nach Anspruch 1 oder 2, wobei die Nichtkulturpflanzenschädlinge aus der Gruppe bestehend aus den Ordnungen Isoptera, Blattaria (Blattodea), Diptera, Hymenoptera, Siphonaptera, Orthoptera und Ixodida stammen.

4. Verwendung der Verbindungen der Formel I gemäß Anspruch 1 zum Schutz von nichtlebenden organischen Substanzen.

5. Verwendung nach Anspruch 4 zum Schutz von nichtlebenden organischen Substanzen gegen Nichtkulturpflanzenschädlinge aus der Gruppe bestehend aus der Klasse Diplopoda und den Ordnungen Isoptera, Diptera, Blattaria (Blattodea), Dermaptera, Hemiptera, Hymenoptera, Orthoptera und Thysanura.

6. Verfahren nach den Ansprüchen 1 bis 5, wobei es sich bei der Verbindung der Formel I um eine Verbindung der Formel I-1 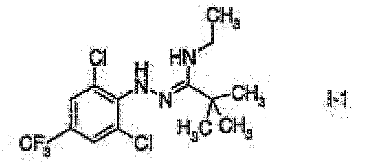 handelt.

7. Verfahren nach den Ansprüchen 1 bis 5, wobei es sich bei der Verbindung der Formel 1 um eine Verbindung der Formel I-2 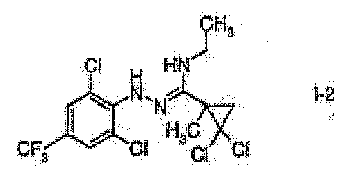 handelt.

8. Verfahren zur Bekämpfung von Nichtkulturpflanzenschädlingen, bei dem man die Schädlinge oder ihre Nahrungsquelle, ihren Lebensraum, ihre Brutstätten oder den Ort ihres Auftretens mit einer pestizidwirksamen Menge einer Verbindung der Formel I gemäß den Ansprüchen 1, 6 oder 7 in Kontakt bringt.

9. Verfahren nach Anspruch 8, wobei die Nichtkulturpflanzenschädlinge aus der Gruppe bestehend aus den Klassen Chilopoda und Diplopoda und den Ordnungen Isoptera, Diptera, Blattaria (Blattodea), Dermaptera, Hemiptera, Hymenoptera, Orthoptera, Siphonaptera, Thysanura, Phthiraptera, Araneida, Parasitiformes und Acaridida stammen.

10. Verbindung nach Anspruch 8 oder 9, wobei die Nichtkulturpflanzenschädlinge aus der Gruppe bestehend aus den Ordnungen Isoptera, Blattaria (Blattodea), Diptera, Hymenoptera, Siphonaptera, Orthoptera und Ixodida stammen.

11. Verfahren Bekämpfung von nichtlebenden organischen Substanzen gegen Nichtkulturpflanzenschädlinge aus der Gruppe bestehend aus der Klasse Diplopoda und den Ordnungen Isoptera, Diptera, Blattaria (Blattodea), Dermaptera, Hemiptera, Hymenoptera, Orthoptera und Thysanura, bei dem man die Schädlinge oder ihre Nahrungsquelle, ihren Lebensraum, ihre Brutstätten oder den Ort ihres Auftretens mit einer pestizidwirksamen Menge einer Verbindung der Formel I gemäß den Ansprüchen 1, 6 oder 7 in Kontakt bringt.

12. Verfahren zum Schutz von Tieren gegen Nichtkulturpflanzenschädlinge aus der Gruppe bestehend aus der Klasse Chilopoda und den Ordnungen Araneida, Hemiptera, Diptera, Phthiraptera, Siphonaptera, Parasitiformes und Acaridida, bei dem man die Schädlinge in Gewässern und/oder in und um Gebäude mit einer pestizidwirksamen Menge einer Verbindung der Formel I nach den Ansprüchen 1, 6 oder 7 in behandelt.

13. Verfahren nach Anspruch 12, wobei die Nichtkulturpflanzenschädlinge aus der Gruppe der Ordnungen Diptera, Phthiraptera, Siphonaptera und Parasitiformes stammen.

14. Köderzusammensetzung, die eine pestizidwirksame Menge einer Verbindung der Formel I gemäß den Ansprüchen 1, 6 oder 7 sowie ein Attraktans enthält.

## Revendications

1. Utilisation de composés de formule 1 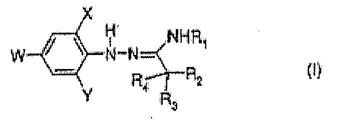 dans laquelle
W est du chlore ou du trifluorométhyle;
X et Y sont chacun indépendamment du chlore ou du brome;
R1 est un alkyle en C₁-C₆, un alcényle en C₃-C₆, un alcynyle en C₃-C₆, ou
un cycloalkyle en C₃-C₆ qui peut être substitué avec 1 à 3 atomes d'halogène ou
un alkyle en C₂-C₄ qui est substitué par un alcoxy en C₁-C₄ ;
R2 et R3 sont un alkyle en C₁-C₆ ou peuvent former conjointement un cyclalkyle en C₃-C₆ qui peut être non substitué ou substitué par 1 à 3 atomes d'halogène;
R4 est un hydrogène ou un alkyle en C₁-C₆,
ou des énantiomères ou des sels de ceux-ci,
pour lutter contre les parasites n'attaquant pas les cultures.

2. Utilisation selon la revendication 1, dans laquelle les parasites n'attaquant pas les cultures sont sélectionnés parmi le groupe composé des classes Chilopoda et Diplopoda et des ordres Isoptera, Diptera, Blattaria (Blattodea), Dermaptera, Hemiptera, Hymenoptera, Orthoptera, Siphonaptera, Thysanura, Phthiraptera, Araneida, Parasitiformes et Acaridida.

3. Utilisation selon les revendications 1 ou 2, dans laquelle les parasites n'attaquant pas les cultures sont sélectionnés parmi le groupe composé des ordres Isoptera, Blattaria (Blattodea), Diptera, Hymenoptera, Siphonaptera, Orthoptera et Ixodida.

4. Utilisation des composés de formule 1 tels que définis à la revendication 1 pour la protection des matières organiques non vivantes.

5. Utilisation selon la revendication 4 pour la protection de matières organiques non vivantes contre les parasites n'attaquant pas les cultures sélectionnés parmi la classe Diplopoda et les ordres Isoptera, Diptera, Blattaria (Blattodea), Dermaptera, Hemiptera, Hymenoptera, Orthoptera et Thysanura.

6. Utilisation selon les revendications 1 à 5, dans laquelle le composé de formule I est un composé de formule I-1.

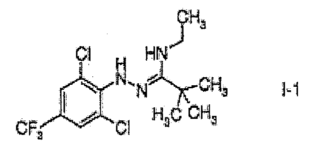

7. Utilisation selon les revendications 1 à 5, dans laquelle le composé de formule I est un composé de formule I-2.

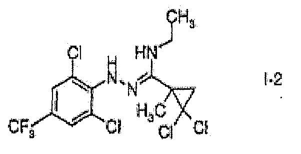

8. Procédé de contrôle des parasites n'attaquant pas les cultures par mise en contact des parasites ou de leur nourriture, leur habitat, leurs terrains de reproduction ou leur lieu avec une quantité efficace sur le plan pesticide d'un composé de formule I tel que défini selon les revendications 1, 6 ou 7.

9. Procédé selon la revendication 8, dans laquelle les parasites n'attaquant pas les cultures sont sélectionnés parmi le groupe composé des classes Chilopoda et Diplopoda et des ordres Isoptera, Diptera, Blattaria (Blattodea), Dermaptera, Hemiptera, Hymenoptera, Orthoptera, Siphonaptera, Thysanura, Phthiraptera, Araneida, Parasitiformes et Acaridida.

10. Procédé selon les revendications 8 ou 9, dans laquelle les parasites n'attaquant pas les cultures sont sélectionnés parmi le groupe composé des ordres Isoptera, Blattaria (Blattodea), Diptera, Hymenoptera, Siphonaptera, Orthoptera et Ixodida.

11. Procédé pour la protection des matières organiques non vivantes contre des parasites n'attaquant pas les cultures sélectionnés parmi le groupe composé de la classe Diplopoda et des ordres Isoptera, Diptera, Blattaria (Blattodea), Dermaptera, Hemiptera, Hymenoptera, Orthoptera et Thysanura, par mise en contact des parasites ou de leur nourriture, leur habitat, leurs terrains de reproduction, leur lieu ou des matières organiques non vivantes avec une quantité efficace sur le plan herbicide d'un composé de formule I tel que défini selon les revendications 1, 6 ou 7.

12. Procédé de protection d'animaux contre les parasites n'attaquant pas les cultures sélectionnés parmi le groupe composé de la classe Chilopoda et des ordres Araneida, Hemiptera, Diptera, Phthiraptera, Siphonaptera, Parasitiformes et Acaridida, comprenant le traitement des parasites dans des plans d'eau et/ou dans et autour de constructions avec une quantité efficace sur le plan pesticide d'un composé de formule I tel que défini selon les revendications 1, 6 ou 7.

13. Procédé selon la revendication 12, dans laquelle les parasites n'attaquant pas les cultures sont sélectionnés parmi le groupe composé des ordres Diptera, Phthiraptera, Siphonaptera et Parasitiformes.

14. Composition d'amorce contenant une quantité efficace sur le plan pesticide d'un composé de formule I tel que défini selon les revendications 1, 6 ou 7 ainsi qu'un appât.
